# EUROPEAN PATENT SPECIFICATION

(11) **EP 3 500 189 B1**
(45) Date of publication and mention of the grant of the patent: **14.07.2021**
(21) Application number: 17764441.6
(22) Date of filing: 18.08.2017
(51) Int. Cl.: A61B 17/16

(54) **OFFSET REAMER DRIVER**
VERSETZTER ANTRIEB FÜR FRÄSER
ACTIONNEUR EN DÉPORT POUR ALÉSOIR

(30) Priority: 18.08.2016 WO PCT/IB2016/001143; 18.11.2016 US 201615355151; 09.12.2016 US 201662431908 P
(43) Date of publication of application: 26.06.2019
(73) Proprietor: Incipio Devices SA, 2072 St-Blaise (CH)
(72) Inventor: CHENAUX, Fabrice, 2016 Cortaillod (CH); LECHOT, André, 2534 Orvin (CH); GENTIL, Thierry, 2533 Evilard (CH)
(74) Representative: Mötteli-Mantelli, Novella
(86) International application number: PCT/IB2017/000988
(87) International publication number: WO 2018/033788

(56) References cited:
- US-A1- 2007 073 302
- US-A1- 2008 058 804
- US-A1- 2013 331 841

## Description

### Cross Reference to Related Applications

U.S. Provisional Application No. 62/431,908, filed December 9, 2016, entitled OFFSET REAMER DRIVER, and having publication US2018/0049753, U.S. Regular Application No 15/355,151, filed November 18, 2016, entitled REAMER DRIVER CONNECTION and having publication No. US10,660,658B2 as well as PCT/IB2016/001143, filed August 18, 2016, entitled OFFSET REAMER DRIVER and having publication No. WO/2017/029546, are related publications.

### Background of the Invention

This invention relates to a surgical reamer driver suitably constructed to be used to reshape the acetabulum. In particular, it relates to surgical reamer drivers that are sealed and made up of few components.

Such surgical reamer drivers exist. For example, US patent application serial no. 11/536,792 to Lechot and having publication US7749227B2, describes a surgical reamer driver having essentially five components, namely 1) a first housing shell, 2) a second housing shell which in a clam-shell-like fashion may be justapoxed against the first housing shell, 3) a transmission drive to be enclosed between the two housing shells, the drive having at least one universal joint and a surgical tool connector at the distal end thereof, 4) a motor shaft coupling at the proximal end thereof and 5) a handle assembly.

Further, these basic components form a surgical reamer driver which in a fully assembled state effectively prevents debris from access in the inner workings of the surgical reamer driver, but they do not guarantee a near perfect sealing due largely to the long, near flat surfaces of the housing shells which can almost never be fully sealed.

A surgical reamer driver is provided in US published patent application serial No. US2013/331841A1 to Douglas. In particular, this document discloses a method for disassembling a reamer driver including the steps of:
a. actuating a sliding release sleeve to unlock a handle assembly from a housing assembly, thereby permitting the de-encapsulation of a drive train within the housing assembly;
b. sliding the handle assembly off of the housing thereby effectively de-encapsulating the drive train;
c. pulling a motor shaft coupling out of the housing thereby freeing the drive train from axial constraint on one end;
d. unsnapping the drive train on the one end and lifting the one end out of the housing assembly thereby permitting removal of the drive train; and
e. pulling the drive train out of the housing assembly, thus removing the drive train from the housing assembly.

A prior art surgical reamer driver is also provided in US patent application publication US2008/0058804 to Lechot et al. This document discloses a surgical reamer driver having a housing assembly in a stand-alone, assembled unit, a transmission drive train in the housing assembly, with universal joints, and with a surgical tool connector at its distal end and a motor shaft coupling at its proximal end, and a handle assembly in a stand-alone, assembled unit disposed over the housing assembly.

There exists a need for a surgical reamer driver in order to avoid penetration of debris and abrasion of soft tissues into the mechanism of the said surgical reamer driver.

### Summary of the Invention

An improved surgical reamer driver has four basic components and a distal and proximal end. The four components include a housing assembly, a transmission drive train, a motor shaft coupling, and a handle assembly. The transmission drive train is enclosed in the housing assembly, and has a surgical tool connector at the distal end thereof. The motor shaft coupling is disposed at the proximal end thereof. The handle assembly is disposed at the proximal end thereof.

An object of the invention is to provide a surgical reamer driver which, in a fully assembled state, effectively prevents debris from access in the inner workings of the device. This encapsulation of the inner workings also prevents abrasion of soft tissues during use.

Another object of the invention is to provide a surgical reamer driver which allows an easy replacement of components e.g. when components are worn out.

Another object of the invention is to provide a transmission drive train having at least a double universal joint linkage (unlike a normal U-joint having only one knuckle, a double U-Joint has two separate knuckles at different spaced relationships along the shaft on which the joints are disposed) that can transmit rotational movement at an angle larger than 40°. The two forks of the outermost universal joint linkages (those which are most widely spaced apart from each other) are oriented at 90° from each other.

Another object of the invention is to provide a housing assembly that can enclose the transmission drive train and maintain it in place with a set of bearings disposed along the train.

Another object of the invention is to provide a surgical reamer driver wherein the transmission of the load applied on the motor shaft coupling is essentially transmitted to the body of the housing assembly. The load applied on the handle is also essentially transmitted to the body of the housing assembly. There is no contact between the motor shaft coupling and the handle assembly. These two cumulated loads are directly transmitted to the quick tool connector without compressing the universal joint transmission drive train, which transmits the torque applied essentially on the motor shaft coupling.

Another object of the invention is to provide a surgical reamer driver having a simple surgical reamer driver connection that allows quick connect of different types of acetabular reamers from the center of the surgical reamer driver with a mechanism with no nukes or crannies that might trap or attract bone chips or debris. In comparison to the existing surgical reamer driver connections described in the prior art, the locking mechanism located in the center of the surgical reamer driver consists of a plate whose length in the axial direction allows for axial translation without revealing spaces in which debris or chips might enter, thereby preventing such debris and bone chips from jamming the mechanism. Chips and debris is highly undesirable as such may potentially disconnect the reamer from the surgical reamer driver. It also reduces soft tissue irritation while rotating by limiting the sharp edges of components located around the quick tool connector of the surgical reamer driver. Another object of the invention is to provide a locking mechanism in the quick tool connector of a surgical reamer driver which, unlike the standard lock/release function, can be locked in its open position. This allows the surgeon to insert the cutting tool through a minimal invasive opening first. Then, once locked, the reamer driver can be inserted through the same minimal invasive opening and connected to the cutting tool without activating the locking of the mechanism.

### Brief Description of the Drawings

The attached drawings represent, by way of example, different embodiments of the subject of the invention.
FIG. **1** is a perspective view of the fully assembled surgical reamer driver.
FIG. **2** is an exploded view of main components of the surgical reamer driver.
FIG. **3A** is a bottom view of the housing assembly of the surgical reamer driver, showing the transmission drive train assembled.
FIG. **3B** is a perspective view of the housing assembly of the surgical reamer driver, showing the transmission drive train assembled.
FIG. **4** is a perspective view of the housing assembly of the surgical reamer driver, showing a part of the transmission drive train, and showing the motor shaft coupling uncoupled from the transmission drive train.
FIG. **5A** is a perspective view of the housing assembly of the surgical reamer driver, showing a part of the transmission drive train, and showing the motor shaft coupling coupled to the transmission drive train.
FIG. **5B** is a detail of FIG. **5A** showing the motor shaft coupling in the position coupled to the transmission drive train.
FIG. **6** is a perspective view of the housing assembly of the surgical reamer driver, showing a part of the transmission drive train, the motor shaft coupling coupled to the transmission drive train, and the handle assembly uncoupled to the motor shaft coupling and uncoupled to the housing assembly of the surgical reamer driver.
FIG. **7** is a cross-section of the fully assembled surgical reamer driver.
FIG. **8** is a detail of FIG. **7** showing interconnection between the handle assembly, the housing assembly, the motor shaft coupling and the transmission drive train.
FIG. **9** is a detail of FIG. **7** showing the quick tool connector.
FIG. **10** is a detail of FIG. **7** showing the proximal portion of the transmission drive train positioned in the body of the housing assembly of the surgical reamer driver.
FIG. **11A** is a detail of FIG. **7** showing the distal portion of the transmission drive train positioned in the body of the housing assembly of the surgical reamer driver.
FIG. **11B** is a cross-section of FIG. **11A** showing the transmission drive train maintained in position by a set of bearings.
FIG. **12** is a detailed exploded view of individual components used in a variant of the invention.
FIG. **13** is a detailed exploded view of typical components used on a universal joint as used as functional element(s) of the transmission drive train.
FIG. **14** is a perspective view of a universal joint as used as functional element(s) of the transmission drive train.
FIG. **15** is a detailed exploded view of the quick tool connector.
FIG. **16** is a perspective view of the quick tool connector.
FIG. **17** is a partially exploded view of the quick tool connector.
FIG. **18** is a perspective view of the quick tool connector connection in the lock open position.
FIG. **19** is a multiple reamer coupling.
FIG. **20** is a multiple reamer coupling.
FIG. **21** is a multiple reamer coupling.
FIG. **22** is a kit comprising the invention.
FIG. **23** is a flow chart of the method of using the invention.

Those skilled in the art will appreciate that elements in the Figures are illustrated for simplicity and clarity and have not necessarily been drawn to scale. For example, dimensions may be exaggerated relative to other elements to help improve understanding of the invention and its embodiments. Furthermore, when the terms 'first', 'second', and the like are used herein, their use is intended for distinguishing between similar elements and not necessarily for describing a sequential or chronological order. Moreover, relative terms like 'front', 'back', 'top' and 'bottom', and the like in the Description and/or in the claims are not necessarily used for describing exclusive relative position. Those skilled in the art will therefore understand that such terms may be interchangeable with other terms, and that the embodiments described herein are capable of operating in other orientations than those explicitly illustrated or otherwise described.

### Detailed Description of the Preferred Embodiment(s)

The following description is not intended to limit the scope of the invention in any way as they are exemplary in nature, serving to describe the best mode of the invention known the inventors as of the filing date hereof.

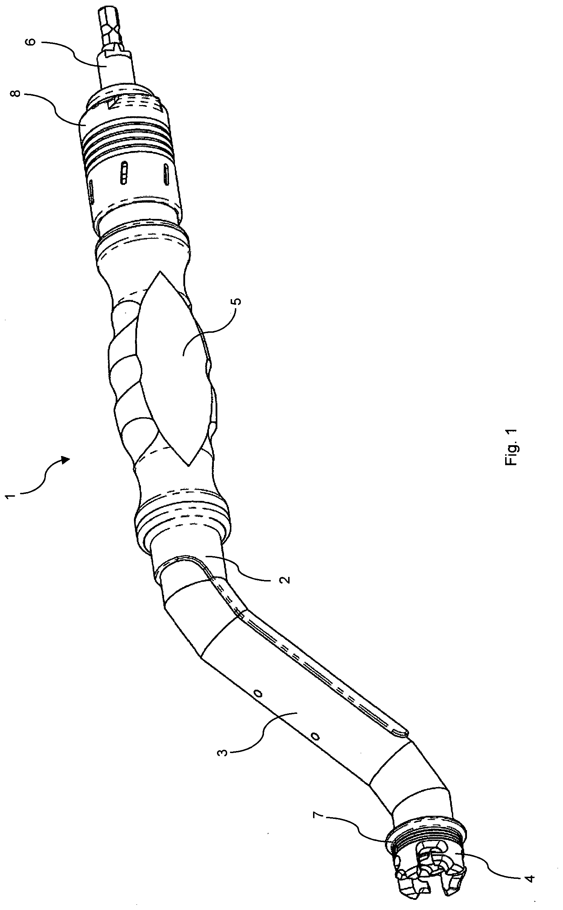

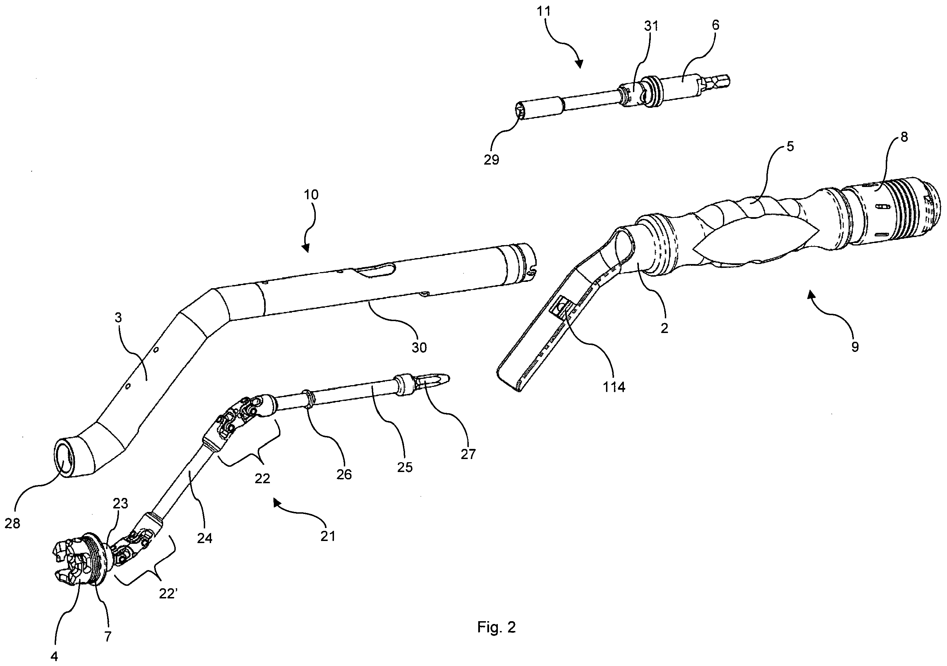

Referring to FIG. **1** showing the assembled surgical reamer driver 1. Such a surgical reamer driver 1 is a surgical instrument used to drive bone cutting tools during minimal invasive surgeries. A partially open housing portion 2 of the handle assembly 9 fully closing the bottom opening 30 of the housing assembly 10. This partially open housing portion 2 (similar to tube 2 of PCT/IB2016/001143, having publication No. WO/2017/029546) is part of the handle assembly 9 (as with that shown in the above-mentioned WO2017/029546, fig. 2 and the text of the detailed description associated therewith). Housing assembly 10 preferably has a Z shape at the position of the distal body portion 3 of the housing assembly 10, where the central axis of the proximal transmission shaft 25 (power input) and the central axis of the distal transmission shaft 24 (power output) are not coincident. A quick tool connector 4 (such as that described in US patent application no. 15/355,151, having publication No. US 10,660,658B2), is affixed to the distal transmission shaft 24. Bone cutting tools (not shown) are connected to the said quick tool connector 4. Handle 5 is part of handle assembly 9. The handle 5 may be for example out of metal, plastic or silicone, and possess an anti-slippery coating, and may be shaped ergonomically, with or without anti-slippery profile. A motor shaft quick connection 6 allows the application of torque. The ring 7 allows the release of a bone cutting tool (not shown). The release sleeve 8 (as with the same numbered assembly in the above-mentioned WO2017/029546) allows the release of the handle assembly 9. One of the differences to the known prior art is that the device is fully encapsulated, avoiding penetration of debris and abrasion of soft tissues during use.

The variant shown in the figures is made out of four main components, the transmission drive train 21 in a stand-alone, assembled unit (it is held together, mechanically locked, by the interconnection of the components), the housing assembly 10 in a stand-alone, assembled unit, the motor shaft coupling 11 in a stand-alone, assembled unit and the reamer handle 9 in a stand-alone, assembled unit.

Now referring to FIG. 2, the main components, the transmission drive train 21, the housing assembly 10, the motor shaft coupling 11 and the handle assembly 9, separated from each other are shown, as with Fig. 2 of the above-mentioned WO2017/029546.

Mechanical load applied on the handle 5 is transmitted through to the head bearing 23 and finally to the quick tool connector 4. There is no transmission of load into the motor shaft coupling 11. The head bearing 23 may be made for example out of PEEK, carbon fiber PEEK, Teflon, PPSU, metal. The transmission drive train typically includes universal joints 22, 22', a central transmission shaft 24, a proximal transmission shaft 25, a stop ring 26 allowing the axially positioning of the transmission drive train when inserted into the bearing(s) 32, 35, 115 and 115', a rotational transmission feature 27 (Hex, square, triangle, ...) allowing transmission of the rotational torque from the motor shaft coupling 11 to the transmission drive train 21. This rotational transmission feature 27 transmits only rotational torque but not the eventual axial force applied on the motor shaft coupling 11. A distal front opening 28 of the housing assembly 10 where the transmission drive train 21 can be inserted. A rotational transmission feature 29 (Hexagon, square, triangular, or any polygonal shape) to be connected with rotational transmission feature 27. An elongated bottom opening 30 of the housing assembly 10 where the transmission drive train 21 exits during while inserting into the housing assembly 10 and before it reaches its assembled position. Motor shaft bearing 31 may be made for example out of PEEK, carbon fiber PEEK, Teflon, PPSU or metal. One or more distal transmission drive train bearing(s) 32, 35, 115, 115', may be made for example out of PEEK, carbon fiber PEEK, Teflon, PPSU or metal, having a snapping feature to capture the transmission shafts 24, 25 and maintain them in place. This distal transmission drive train bearing(s) 32, 35, 115, 115' are also ensuring the axial positioning of the transmission drive train with the stop ring 26. Another distal transmission drive train bearing 114 is assembled in the distal portion of the partially open housing portion 2 of the handle assembly 9 and secures the transmission drive train in place when the handle assembly 9 is locked onto the housing assembly 10. This is particularly important with this configuration of transmission drive train having two double u-joints linkage (unlike a normal U-joint having only one knuckle, a double U-Joint has two separate knuckles at different spaced relationships along the shaft on which the joints are disposed) because a torque applied on the rotational transmission feature 27 tends to unsnap the transmission drive train from the bearing 115, 115'. The transmission drive train bearing 114 cooperates with the opposite bearings 115, 115' in the distal body portion 3 of the housing assembly 10 to stabilize the drive train 21. The transmission drive train bearing 114 may be made for example out of PEEK, carbon fiber PEEK, Teflon, PPSU or metal.

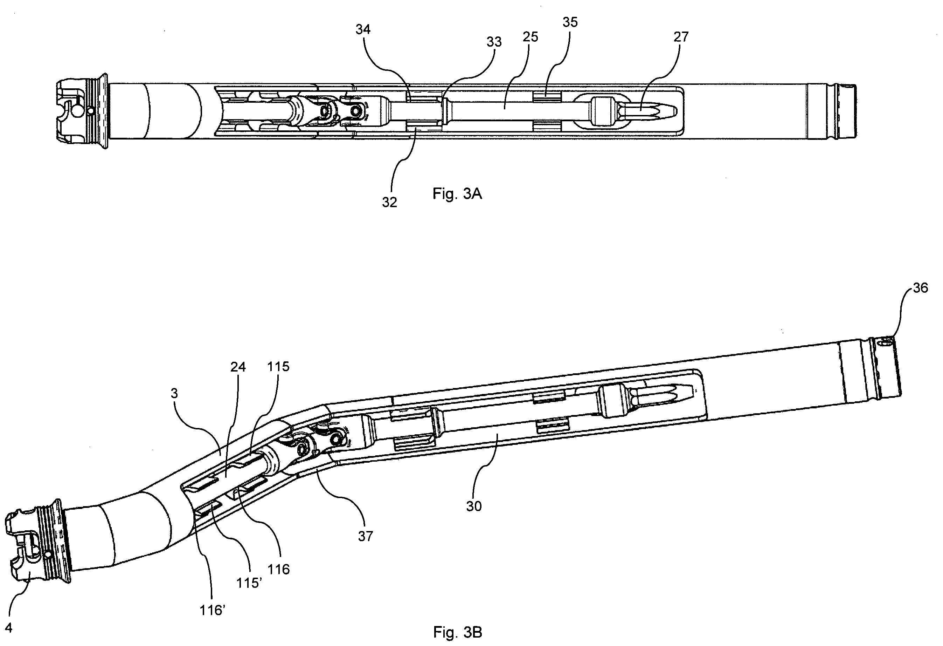

Now referring to FIG. **3A** and FIG. **3B****,** whereof FIG. **3A** is showing a top view of the housing assembly 10 of the surgical reamer driver 1, showing the transmission drive train 21 assembled and FIG. **3B** is showing a perspective view of the housing assembly 10 of the surgical reamer driver 1, showing the transmission drive train assembled. A flange 33 between the stop ring 26 and the distal transmission drive train bearing(s) 32 is avoiding axial frontward movement of the shaft 25. A point of contact 34 between the proximal transmission shaft 25 and the distal transmission drive train bearing(s) 32, 35 ensures the concentricity of the proximal transmission shaft 25 within the housing (e.g. tubes) of the housing assembly 10 and allows its rotation. A point of contact 116 between the central transmission shaft 24 and the distal transmission drive train bearing(s) 115, 115' ensures the concentricity of the central transmission shaft 24 within the housing (e.g. tubes) of the housing assembly 10 and allows its rotation. Groove 36 allowing the angular positioning of the handle in the correct orientation.

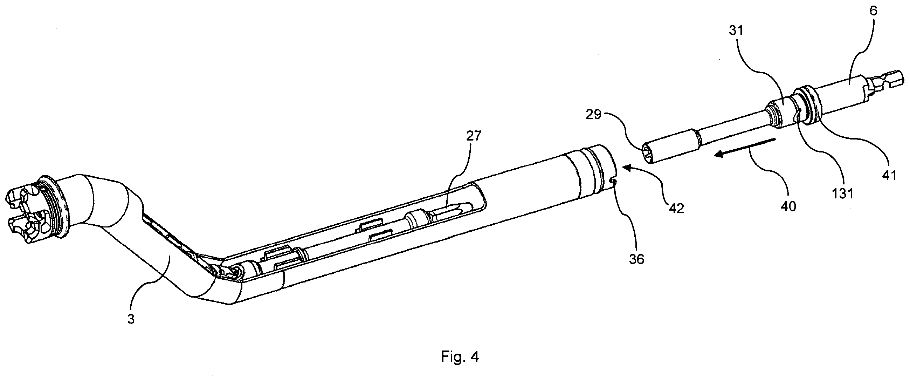

Now referring to FIG. **4** showing a perspective view of the housing assembly 10 of the surgical reamer driver 1, showing a part of the transmission drive train 21, and showing the motor shaft coupling 11 uncoupled from the transmission drive train 21. Insertion of the motor shaft coupling 11 in direction 40 into the housing assembly 10. A retaining ring 41 allowing transmission of the axial load on the motor shaft quick connection 6 onto the motor shaft bearing 31. A proximal back opening 42 of the housing assembly 10 where the motor shaft coupling 11 is being inserted. An elastic member 131 of the motor shaft bearing 31 retains the motor shaft coupling 11 in the housing assembly 10 while permitting rotation.

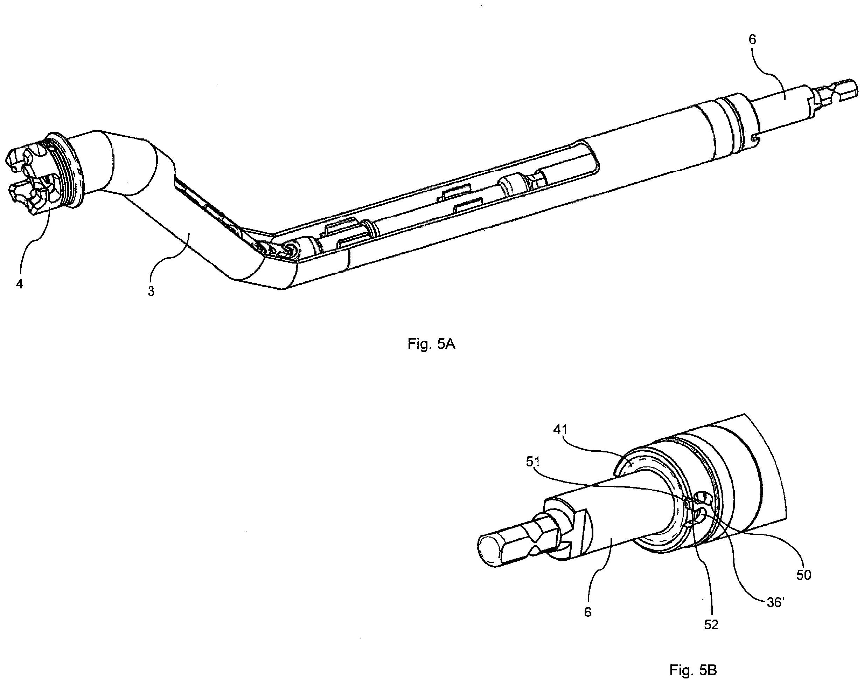

Now referring FIG. **5A** showing a perspective view of the housing assembly 10 of the surgical reamer driver 1, showing a part of the transmission drive train 21, and showing the motor shaft coupling 11 coupled to the transmission drive train 21.

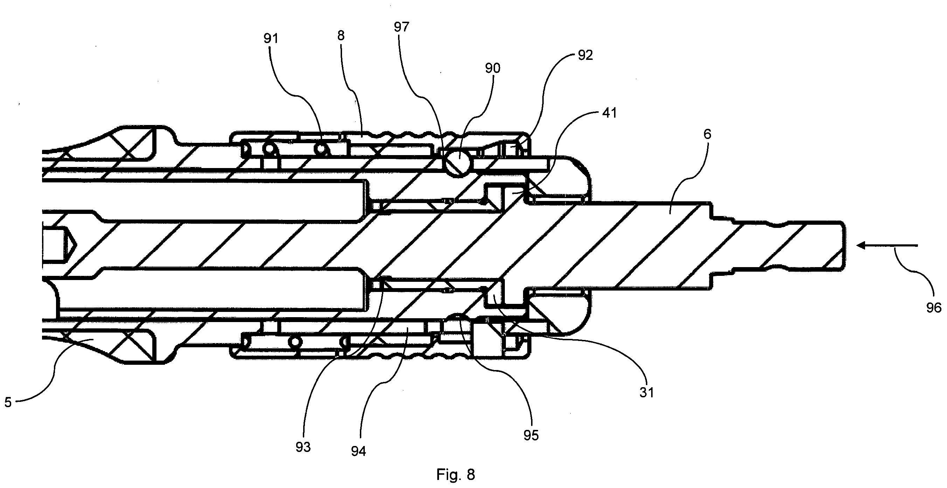

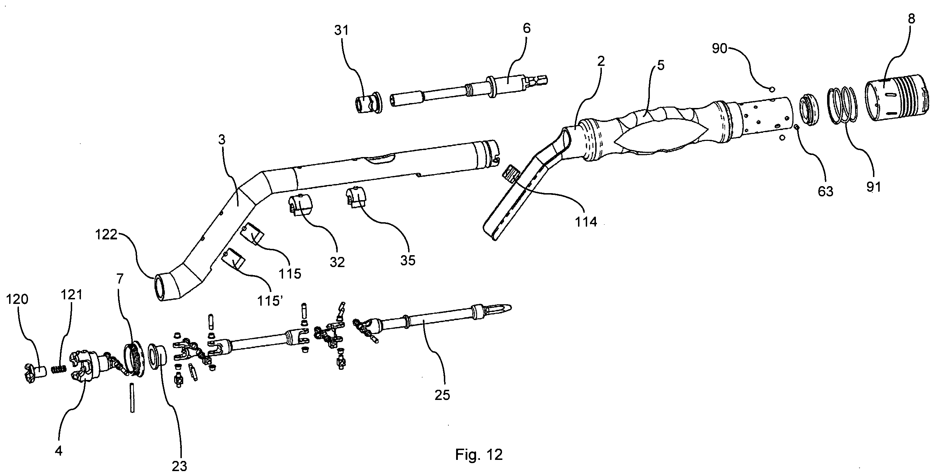

Now referring FIG **5B** showing a detail of FIG. **5A** showing the motor shaft coupling 11 in the position coupled to the transmission drive train. Whereas the transmission of the load 96 (see FIG. 8) applied on the motor shaft coupling 11 is made through the retaining ring 41 to the motor shaft bearing 31 and finally to the housing assembly 10. No load is transmitted into the handle assembly 9. There is no contact between the motor shaft coupling 11 and the handle assembly 9. A snap feature of the invention for retaining the handle 5 against free fall or inadvertent release when disassembling the reamer driver. The snap feature is enabled by creating a flexible finger retention via, for example, an adjacent relief slot 50 to pin recesses 36', which enables a raised boss 52 on a finger 51 created by this adjacent slot, to move out of the way of an annular locking feature, such as a pin 63 (shown in FIG. 12), and snap back thereby retaining the handle. At least one annular locking feature, such as a pin 63 located in the handle assembly 9 form-locks with at least one of the grooves 36 and allows the angular positioning of the handle 5.

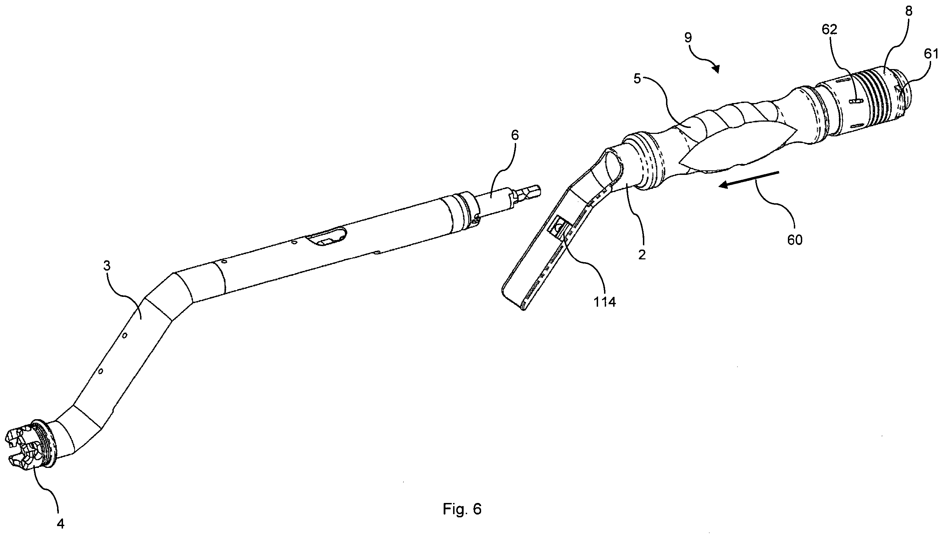

Now referring to FIG. **6** showing a perspective view of the housing assembly 10 of the surgical reamer driver 1, showing a part of the transmission drive train 21, the motor shaft coupling 11 coupled to the transmission drive train 21, and the handle assembly 9 uncoupled to the motor shaft coupling 11 and uncoupled to the housing assembly 10 of the surgical reamer driver 1. Insertion of the handle assembly 9 in direction 60 onto the assembled housing assembly 10, transmission drive train 21 and motor shaft coupling 11. A trigger feature 61 of the release sleeve 8 allowing release of the handle assembly 9. This trigger feature 61 can be made longer in order to be activated by e.g. a finger without moving the hand away from the handle 5 (similar a trigger of a pistol). One or more openings 62 into the release sleeve 8 allowing circulation of water/steam during cleaning and sterilization.

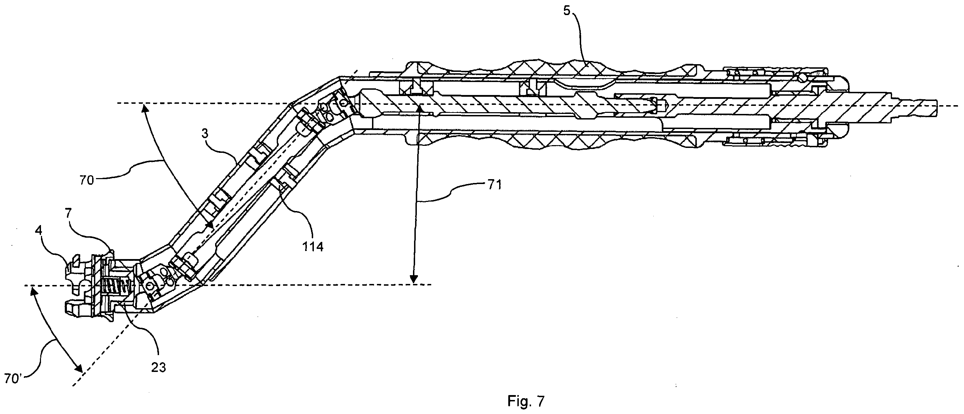

Now referring to FIG. 7 showing a cross-section of the fully assembled surgical reamer driver 1. The transmission drive train has at least a double universal joint linkage 22 that can transmit rotational movement at an angle 70 larger than 40°. The two forks (131, 131') of the outermost universal joint linkages are oriented at 90° from each other to avoid a dead point while rotating. In the first embodiment of the invention, the input shaft of the transmission drive train 21 is offset by a distance 71 from the output shaft. Both input and output shafts are parallel to each other (the angles 70 and 70' are identical). In a different embodiment, there might only be one double universal joint linkage 22 between the input shaft and the output shaft, wherein the input and output shafts are angled from each other.

Now referring to FIG. **8** showing a detail of FIG. 7 showing interconnection between the handle assembly 9, the housing assembly 10, the motor shaft coupling 11 and the transmission drive train 21. A ball 90 allowing connection of the handle assembly 9 with the housing assembly 10. The at least one ball 90, pushed down by profiled groove 92 of the release sleeve 8, falls into the groove 95.

A spring 91 maintaining the release sleeve 8 in its frontward position. The profiled groove 92 allowing the ball 90 to move away from the groove 95 when the release sleeve 8 is in its backward position and allowing the ball 90 to be pushed into the groove 95 when the release sleeve 8 is in its frontward position in order to lock the handle assembly 9 into the housing assembly 10. A groove 93 on the motor shaft coupling 11 where the lip of the motor shaft bearing 31, slightly smaller in diameter, falls into in order to secure the assembly of the motor shaft bearing 31 onto the motor shaft coupling 11. The groove 95 is formed into the proximal portion 94 of the housing assembly 10. One or more hole(s) 97 are formed through the partially open housing portion 2 where they receive the locking ball(s) 90. The inside edge of the hole(s) 97 (towards the inside of the partially open housing portion 2) has a lip slightly smaller diameter than the hole(s) 97 in order to retain the ball(s) 90 of going out.

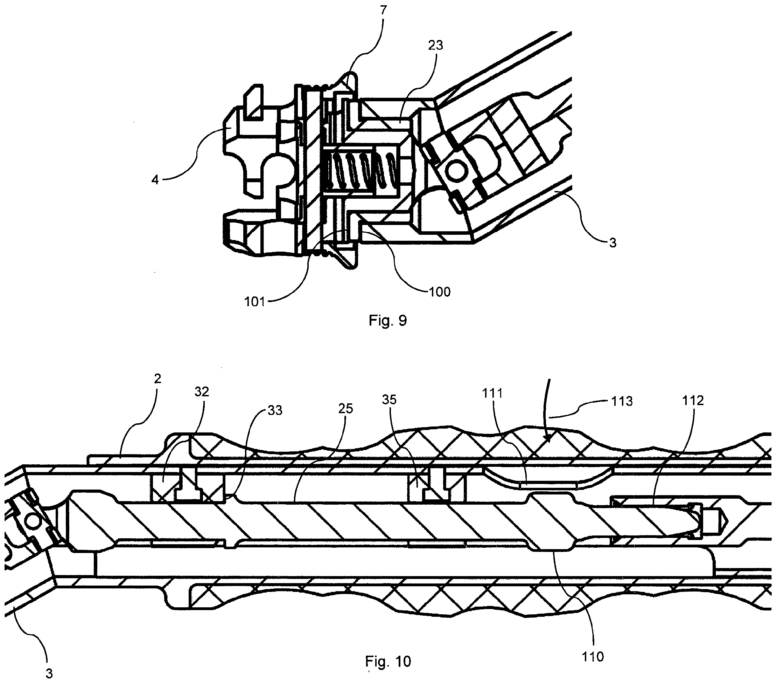

Now referring to FIG. **9** showing a detail of FIG. 7, showing a point of contact 100 between the distal end of the distal body portion 3 (as with that shown in WO2017/029546, fig. 10 and the text of the detailed description associated therewith) and the head bearing 23. An axial load applied on the housing assembly 10 (through the handle assembly 9 and through the motor connection shaft 11) is transmitted to the reamer quick tool connector 4 through the head bearing 23. Further FIG. **9** shows a point of contact 101 between the head bearing 23 and the reamer quick tool connector 4.

Now referring to FIG. **10** showing another detail of FIG. **7****,** showing an enlarged diameter portion 110 of the proximal transmission shaft 25, increasing the surface of contact when pushing the proximal transmission shaft 25 up for disassembling. A distal opening 111 in the housing assembly 10 allowing access with a finger to push the enlarged diameter portion 110 of the proximal transmission shaft 25 up. A point of contact 112 of the rotational transmission feature 27 allowing transmission of the rotational torque from the motor shaft coupling 11 to the transmission drive train 21. This feature transmits only rotational torque but not the eventual axial force applied on the motor shaft coupling 11. An access 113 with a finger or other mean to push the proximal transmission shaft 25 up.

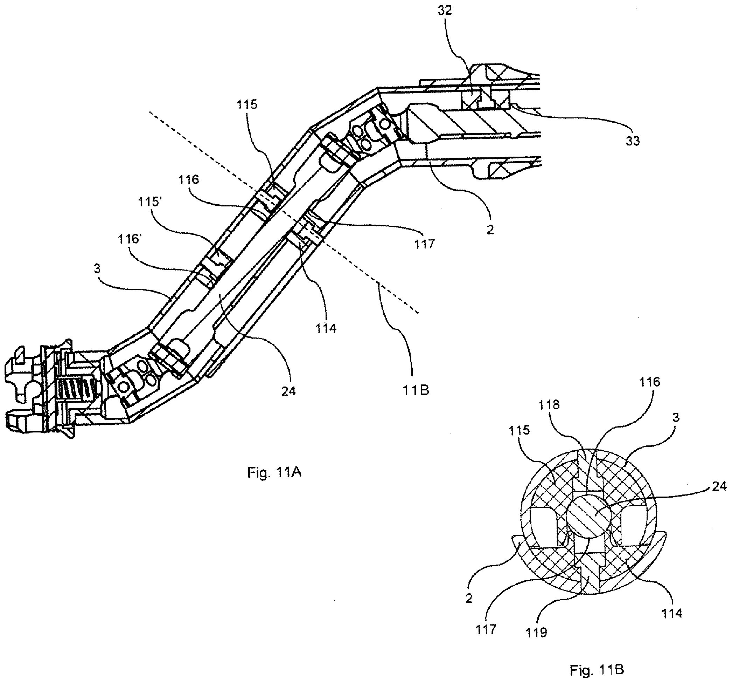

Now referring to FIG. **11A** showing another detail of FIG. 7, showing the central portion of the transmission drive train 21 assembled into the drive train bearings 115, 115' of the housing assembly 10. Points of contact 116, 116' between the central transmission shaft 24 and the distal transmission drive train bearings 115, 115' ensures the concentricity of the central transmission shaft 24 within the housing (e.g. tubes) of the housing assembly 10 and allows its rotation. An optionally distal transmission drive train bearing 114 is assembled in the distal portion of the partially open housing portion 2 and secures the transmission drive train with at a point of contact 117 when the handle assembly 9 is locked onto the housing assembly 10. This distal transmission drive train bearing 114 also ensures correct snapping of the central portion of the transmission drive train 21 into the bearings 115, 115'. In case of incorrect assembling by the user of the device, the drive train bearing 114 will push and snap the central portion of the transmission drive train 21 into the bearings 115, 115' when the handle assembly 9 is locked onto the housing assembly 10.

Now referring to FIG. **11B** showing a cross-section view 11B of FIG. **11A****,** showing the transmission drive train bearings 114, 115 ensuring concentricity of the central transmission shaft 24 within the housing assembly 10. The transmission drive train bearings 114 are assembled to the partially open housing portion 2 with a press-fit and/or welded pin 119. The transmission drive train bearings 116 are assembled to the distal body portion 3 with a press-fit and/or welded pin 118.

Now referring to FIG. **12** showing a detailed exploded view of individual components used in a variant of the invention. A central cutting tool connection 120, a spring 121, a front face 122 of the housing assembly 10, transmitting the axial load applied on the housing assembly 10 (through the handle assembly 9 and through the motor connection shaft 11) to the quick tool connector 4 through the head bearing 23.

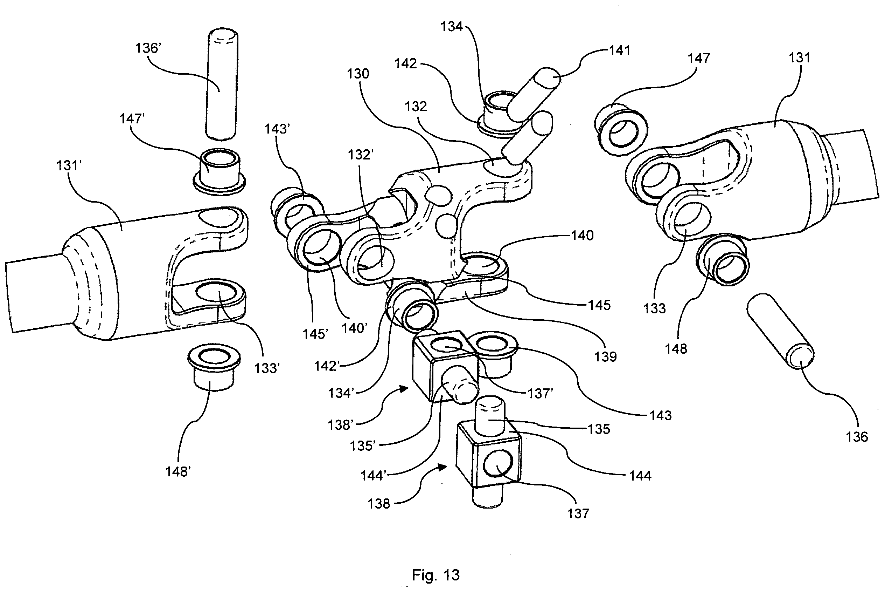

Now referring to FIG. **13** showing a detailed exploded view of typical components used in a double universal joint 22 as used as functional element(s) of the transmission drive train 21. A first half 130 and a second half 139 of the first and second forks of the double universal joint 22. The first and second forks are split in half 130, 139 to allow assembling of the central blocks 138, 138'. In a different embodiment, the first and second forks (130, 139) may be split in any number of ways, for example, in multiple pieces as long as the splits allow assembling of the central blocks 138. A third fork 131 and a fourth fork 131' of the universal joint 22. Bearing surfaces 132, 132' of the first half 130 of the first and second forks. A bearing surface 133 of the third fork 131. Bearing sleeves 134, 134', the bearing sleeves 134, 134' might be made for example out of PEEK, carbon fiber PEEK, Teflon, PPSU or metal. Cylindrical extensions 135 of the central block 138 allowing its rotation with the two halves 130, 139 of the first fork. Cylindrical extensions 135' of the central block 138' allowing its rotation with the two halves 130, 139 of the second fork. A cylindrical pin 136 allowing rotation of central block 138 with the third fork 131. The cylindrical pin 136 is press fit into the central hole 137 of the central block 138. A central hole 137 of the central block 138. A cylindrical pin 136' allowing rotation of central block 138' with the fourth fork 131'. The cylindrical pin 136' is press fit into the central hole 137' of the central block 138'. A central hole 137' of the central block 138'. Bearing surface 140 of the second half 139 of the first fork. Bearing surface 140' of the second half 139 of the second fork. Positioning pins 141, press fit into the two halves 130, 139 to maintain the two halves 130, 139 together. Inner bearing surfaces 142 of the bearing sleeves 134 ensuring positioning and low friction with the inner surfaces 145 of the first fork. Outer bearing surfaces 143 of the bearing sleeves 134 ensuring positioning and low friction with the side surfaces 144 of the central block 138. Side surfaces 144 of the central block 138, adjacent to the cylindrical extension 135. Inner bearing surfaces 142' of the bearing sleeves 134' ensuring positioning and low friction with the inner surfaces 145' of the second fork. Outer bearing surfaces 143' of the bearing sleeves 134' ensuring positioning and low friction with the side surfaces 144' of the central block 138'. Side surfaces 144' of the central block 138', adjacent to the cylindrical extension 135'.

Part of the invention is the use of double universal joints 22 having eight bearing sleeves 134, 134', 147, 147', 148, 148' for this kind of devices. It is expected to highly increase the life of the universal joint 22 by reducing the friction and therefore the wear. More traditional universal joints have metal on metal friction.

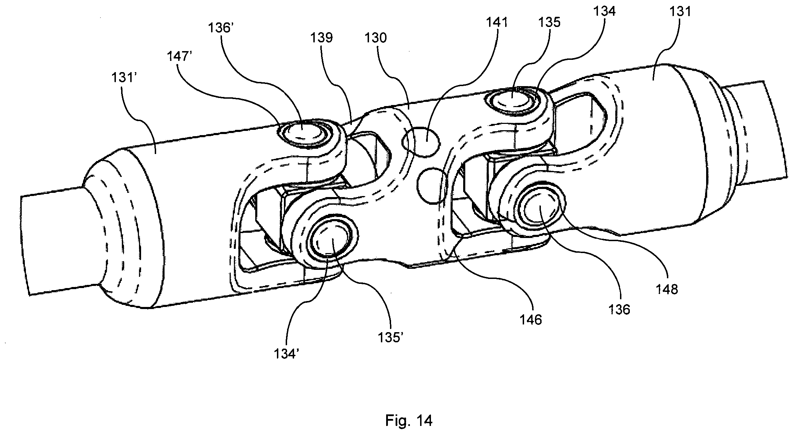

Now referring to FIG. **14** showing a perspective view of a double universal joint 22 as used as functional element(s) of the transmission drive train 21. A contact surface 146 between the two halves 130, 139 of the first and second forks. In addition to the pin 141, the two halves can be secured together by welding, gluing. Another object of this invention is the offset angulation of the first and second fork from each other. The known prior art shows the first and second forks of a double universal joint being in a mirror position from each other, forming an H-shape.

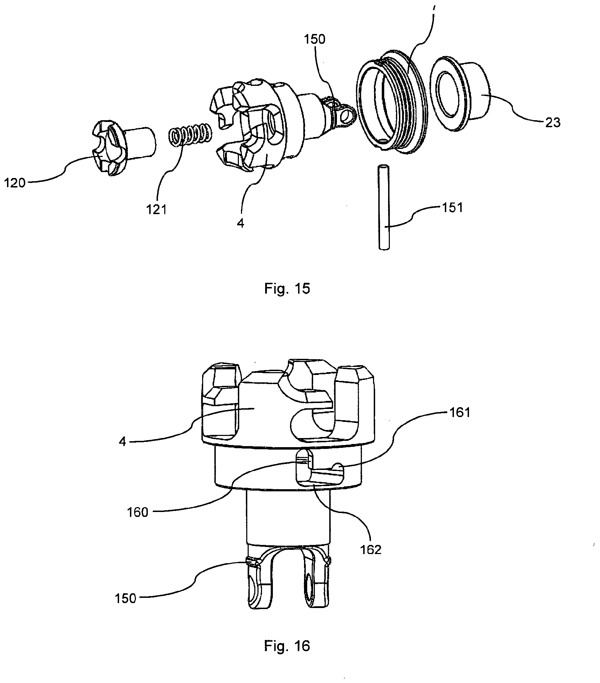

Now referring to FIG. **15** showing a detailed exploded view of quick tool connector 4. A retaining rib 150 allowing the retention of the head bearing 23 once assembled onto the quick tool connector 4. This retaining rib 150 is positioned in such way to allow slight translation movement of the head bearing 23 for easier cleaning but to retain the head bearing 23 of falling off. A pin 151 connecting the ring 7 with the central cutting tool connection 120 together, as also visible in FIG. **10****.**

Now referring to FIG. **16** showing a perspective view of the quick tool connector 4. An elongated groove 160 allowing the cutting tool locking mechanism (ring 7, pin 151 and central cutting tool connection 120) to slide backward/frontward in direction 181 in order to release/lock the cutting tool. The cutting tool locking mechanism is spring loaded with spring 121 in its locked position. The elongated groove 161 allowing the cutting tool locking mechanism (ring 7, pin 151 and central cutting tool connection 120) to be locked in the release (open) position. A channel 162 allowing the cutting tool locking mechanism (ring 7, pin 151 and central cutting tool connection 120) to be switched between the release/lock movement and the locked open position by rotation 180.

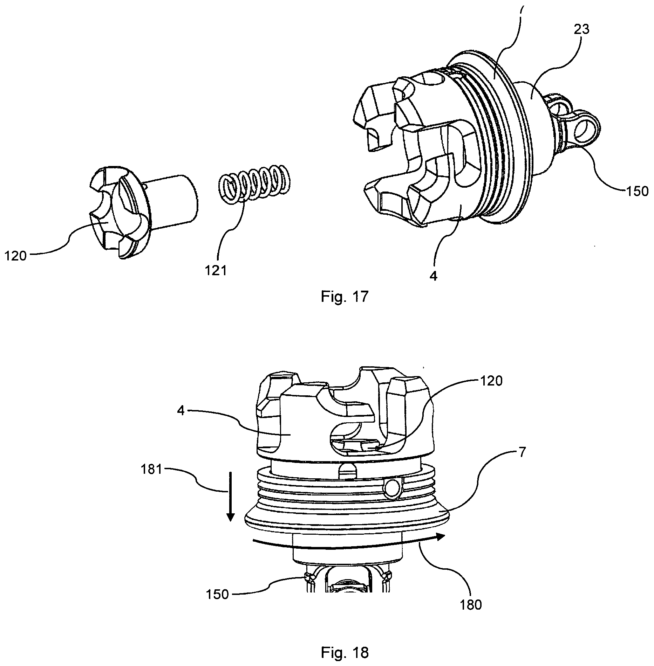

Now referring to FIG. **17** showing a partially exploded view of the quick tool connector 4. A central cutting tool connection 120, a spring 121, a quick tool connector 4.

Now referring to FIG. **18** showing a perspective view of the quick tool connector connection in the lock open position, indicating the direction 180 of the rotation of the ring 7 (and therefore the tool locking mechanism) to switch between the release/lock movement and the locked open position. Indicating the direction 181 of a pull movement of the ring 7 (and therefore the tool locking mechanism) to release the cutting tool.

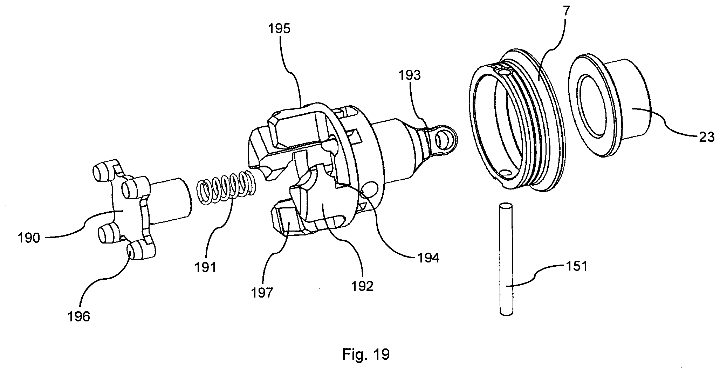

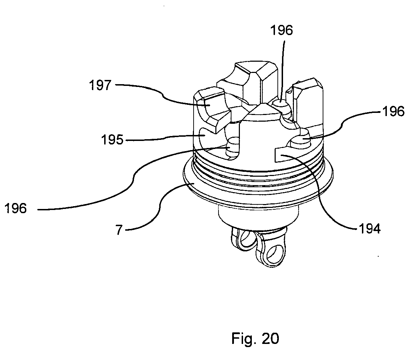

Referring now to FIG. **19** and **20****,** an alternate multiple reamer coupling as quick tool connector of the invention, used to connect with the reamer bar 211 shown in FIG. **30**, has a locking head 190 with at least one pin 196 located in such a way as to close the L-shaped openings 195 and therefore capture the connecting bars of the acetabular reamer once engaged into it in order to maintaining the reamer firmly connected to the surgical reamer driver. Different L-shaped openings 194 may be used to connect non-cylindrical connecting bars of different types of acetabular reamers. As shown is these figures, both rectangular L-shaped openings 194 and cylindrical L-shaped openings 195 are used in the same quick tool connector in order to connect different acetabular reamers having either rectangular or cylindrical connecting bars.

A further alternate embodiment of the multiple reamer coupling of the invention has strategically located pins 196 lock the cutting tool in place.

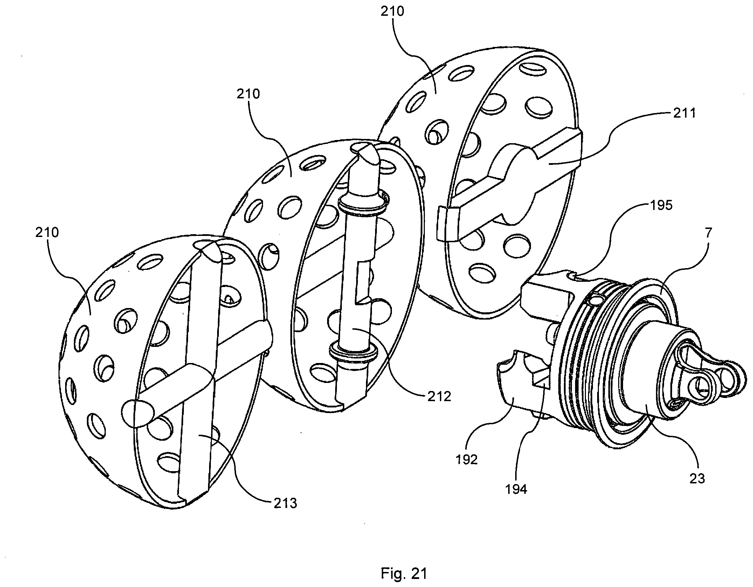

Referring now to FIG. **21****,** the embodiment of FIG. **20** may be configured, based on the location of the locking pins, to lock three different types of tools 210, having three different types of interfaces 211, 212, and 213, respectively.

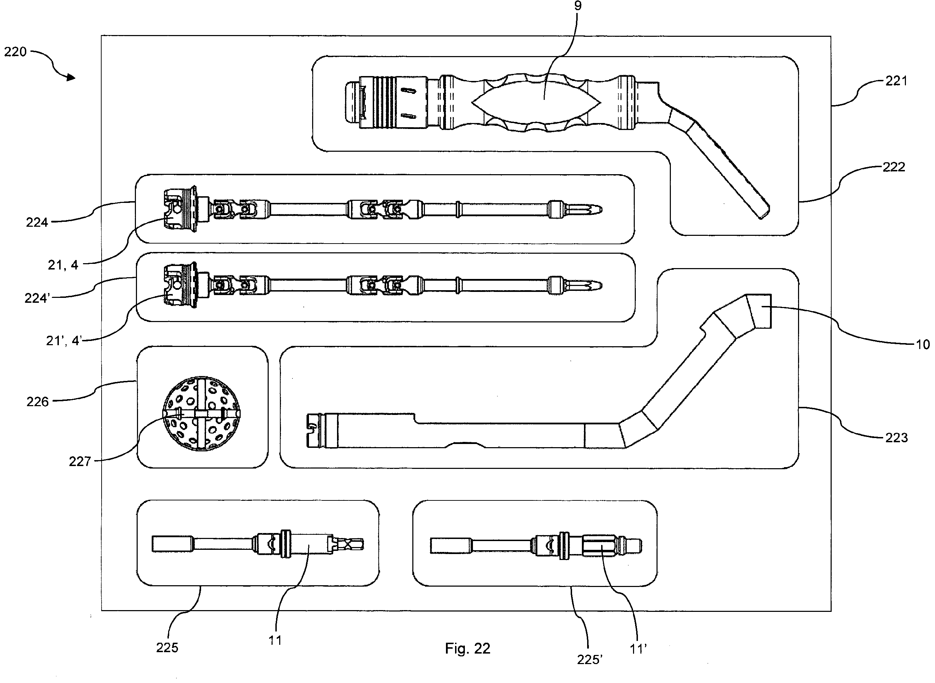

Referring now to FIG. **22** (as with Fig. 31 of WO2017/029546, with reference to the text of the detailed description associated therewith), a kit 220 includes the surgical reamer driver and its components (including some alternate components for alternate configurations), and in addition, a case 221 for organizing and storing the components of the kit. The surgical kit 220 further includes surgical tools 227 (one shown here by duplicates and others having differing outside diameters may be provided) of various sizes and styles, adapted to interface with the surgical tool connector 4. Optionally, alternative motor couplings 11, 11' are provided, each having an alternative connection configuration. Optionally, alternate transmission drive trains 21 and 21' are provided as well, each having an alternate surgical tool connector 4, 4'.

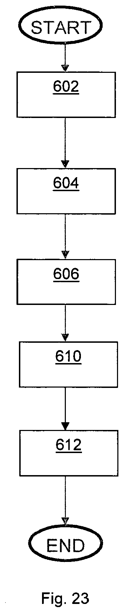

Referring now to FIG. **23****,** the method 600 of using the invention includes several steps. In a first step 602, the sliding release sleeve 8 is actuated to unlock a handle assembly 9 from a housing or housing assembly 10, thereby permitting the de-encapsulation of a drive train 21 within the reamer housing. In a second step 604, the reamer handle is slid off of the housing thereby effectively de-encapsulating the drive train. In a third step 606, the motor shaft coupling 11 is pulled out of the housing thereby freeing the drive train from axial constraint on one end. In a fourth step 610, the drive train is unsnapped on the one end from a restraint 32 and lifted out of the housing thereby permitting removal of the drive train. In a fifth step 612, the drive train is pulled out of the housing, thus removing the drive train from the housing. Once disassembled, the components may be replaced with alternate components meeting another need or simply cleaned and/or sterilized in preparation for the next use.

An advantage of the present invention is to provide a surgical reamer driver having fully closed tube in order to avoid penetration of debris and abrasion of soft tissues during use. The surgical reamer driver shown in this application has only 4 components that can be easily replaced when worn out.

Another advantage of the invention is to provide a transmission drive train having at least a double universal joint linkage that can transmit rotational movement at an angle larger than 40°. The two forks of the universal joint linkage are oriented at 90° from each other.

Another advantage is that the transmission of the load applied on the motor shaft coupling is transmitted to the body of the housing assembly of the reamer driver only. The load applied on the handle is also transmitted to the body of the housing assembly only. There is no contact between the motor shaft coupling and the handle assembly. These two cumulated loads are directly transmitted to the quick tool connector without compressing the universal joint transmission drive train, which only transmit the torque applied on the motor shaft coupling.

An advantage of the present invention is to provide a simple surgical reamer driver connection that allows for the quick connect of different type of acetabular reamers from the center of the surgical reamer driver. In comparison to the existing surgical reamer driver connections described in the prior art, the locking mechanism located in the center of the surgical reamer driver prevent debris and bone chips to enter into the mechanism and potentially disconnect the reamer from the surgical reamer driver. It also reduces soft tissue irritation while rotating by limiting the sharp edges of components located around the quick tool connector of the surgical reamer driver.

In another advantage, the invention provides a locking mechanism in the quick tool connector of a surgical reamer driver which, unlike the standard lock/release function, can be locked in its open position. This allows the surgeon to insert the cutting tool through a minimal invasive opening first. Then, once locked, the reamer handle can be inserted through the same minimal invasive opening and connected to the cutting tool without activating the locking of the mechanism.

Another advantage of the invention is to provide an easy to assemble and disassemble surgical reamer driver connection for better cleaning and sterilization. The number of components and the risk that parts could be lost have been minimized.

It will be understood that the particular devices embodying the invention are shown by way of illustration and not as a limitation of the invention. Although certain illustrative embodiments of the invention have been shown and described here, a wide range of modification, changes and substitutions is contemplated in the foregoing disclosure.

It should be appreciated that the particular implementations shown and herein described are representative of the invention and its best mode and are not intended to limit the scope of the present invention in any way.

The present invention is described herein with reference to block diagrams, devices, components, and modules, according to various aspects of the invention.

The specification and figures should be considered in an illustrative manner, rather than a restrictive one. Accordingly, the scope of the invention should be determined by the appended claims. Further, the elements and/or components recited in apparatus claims may be assembled or otherwise functionally configured in a variety of permutations to produce substantially the same result.

As used herein, the terms "comprises", "comprising", or variations thereof, are intended to refer to a non-exclusive listing of elements, such that any apparatus, article, or composition of the invention that comprises a list of elements, that does not include only those elements recited, but may also include other elements described in the instant specification. Unless otherwise explicitly stated, the use of the term "consisting" or "consisting of" or "consisting essentially of" is not intended to limit the scope of the invention to the enumerated elements named thereafter, unless otherwise indicated. Other combinations and/or modifications of the above-described elements, materials or structures used in the practice of the present invention may be varied or adapted by the skilled artisan to other designs.

Multiple variations and modifications are possible in the embodiments of the invention described here. Although certain illustrative embodiments of the invention have been shown and described here, a wide range of changes, modifications, and substitutions is contemplated in the foregoing disclosure. While the above description contains many specific details, these should not be construed as limitations on the scope of the invention, but rather exemplify one or another preferred embodiment thereof.

## Claims

1. A surgical reamer driver (1) having a distal and proximal end, the surgical reamer driver (1) having:
(a) a housing assembly (10) in a stand-alone, assembled unit having a proximal opening (42) and a distal opening (28),
(b) a transmission drive train (21, 21') in a stand-alone, assembled unit to be enclosed in
the housing assembly (10), and having at least one double universal joint (22, 22') comprising two adjacent universal joints connected with forks (130, 139), and
a surgical tool connector (4) at the distal end,
(c) a motor shaft coupling (11, 11') in a stand-alone, assembled unit to be disposed at the proximal
end thereof, the motor shaft coupling (11, 11') adapted to pass through the proximal
opening (42) and being rotationally connectable and axially, slidably
disengageable from the transmission drive train (21, 21'), and
(d) a handle assembly (9) in a stand-alone, assembled unit to be disposed over the housing
assembly (10) and disengageable from the housing assembly (10),
these four basic components forming a surgical reamer driver (1) which is configured to effectively prevent, in a fully assembled state, debris from access in the inner workings of the surgical reamer driver (1).

2. The surgical reamer driver (1) of claim 1, wherein the at least one double universal joint (22, 22') of the transmission drive train (21, 21') forms an angle (70, 70') larger than 40° between its input and output shafts (24, 25).

3. The surgical reamer driver (1) of claim 1, wherein a bearing (32), fixed with respect to the housing assembly (10) surrounds a flanged shaft (25) having a flange (33), and wherein the flange (33) of the shaft (25) acts against the bearing (32) limiting axial movement of the shaft (25).

4. The surgical reamer driver (1) of claim 1, wherein at least a portion (139) of the forks (130, 139) of the double universal joints (22, 22') may be removed and re-affixed with respect to the other portion (130) of the forks to facilitate assembly.

5. The surgical reamer driver (1) of claim 1, wherein at least one of the forks (130, 139) of the double universal joints (22, 22') comprises friction reducing bearings sleeves (147, 148, 134, 134').

6. The surgical reamer driver (1) of claim 5, wherein the double universal joints (22, 22') have the bearing sleeves (134, 134') assembled in at least one fork (130, 139) thus reducing friction.

7. The surgical reamer driver (1) of claim 1, wherein the handle assembly (9) includes first bearing (114) which, when the handle assembly is assembled over the housing assembly (3, 10) enclosing the drive train (21, 21'), cooperates with second bearings (115, 115') in the housing assembly (3, 10) to stabilize the drive train (21, 21').

8. The surgical reamer driver (1) of claim 1, wherein the at least one double universal joint (22, 22') is comprised of two adjacent universal_joint linkages, each universal joint linkage comprises two forks (131, 139,130, 131'), and the two forks (131, 131') which are most widely spaced apart along the transmission axis are oriented 90° with respect to one another.

9. The surgical reamer driver (1) of claim 1, wherein the transmission drive train (21, 21') comprises two double universal joints (22, 22') having its input and output shafts (24, 25) forming in the assembled configuration an angle (70, 70') larger than 40° and wherein the input shaft (25) of the first double universal joint (22, 22') is offset and parallel to the output shaft (24) of the second double universal joint (22, 22').

10. The surgical reamer driver (1) of claim 1, wherein the motor shaft coupling (11) comprises a first rotational transmission feature (29) on its distal end, the transmission drive train (21) comprises a second rotational transmission feature (27) on its proximal end, wherein in the assembled configuration the first rotational transmission feature (29) and the second rotational transmission feature (27) are axially slidable and radially connected such that rotational movement can be transmitted, wherein in the disassembled configuration, the first rotational transmission feature (29) and the second rotational transmission feature (27) are not connected and the drive train assembly (11, 21) is divided into the motor shaft coupling (11) and the transmission drive train (21), wherein in the assembled configuration the rotational transmission features (29, 27) form a connection between the motor shaft coupling (11) and the surgical tool connector (4) and configured to be covered within the housing assembly (10).

11. The surgical reamer driver (1) of claim 1, wherein the handle assembly (9) is disposed over the housing assembly (10) in the assembled state such that the motor shaft coupling (11, 11') is configured to not transmit axial forces applied by the user to the transmission drive train (21, 21'), and to apply such axial forces to the surgical tool connector (4) and wherein the handle (5) is configured to decouple axial forces applied by the user from both the transmission drive train (21, 21') and the motor shaft coupling (11, 11') and to apply such axial forces to the surgical tool connector (4), preferably through a thrust bearing.

12. The surgical reamer driver (1) of claim 1, wherein the surgical tool connector (4) comprises a central cutting tool connection (120) at its distal end and the motor shaft coupling (11, 11') comprises a motor shaft quick connection (6) at its proximal end, wherein the motor shaft quick connection (6) of the motor shaft coupling (11, 11') is configured to rotatably connect the central cutting tool connection (120) to a drive motor.

13. The surgical reamer driver (1) of claim 1, wherein the surgical reamer driver (1) is configured such that axial forces are transmittable through the housing assembly (10) thereby bypassing the transmission drive train (21, 21'), and transmitted, preferably through a bearing (23), to the surgical tool connector (4).

14. The surgical reamer driver (1) of claim 1, wherein the transmission drive train (21) comprises a rotational transmission feature (27) on its proximal end, wherein the handle assembly (9) includes a release sleeve (8) and a partially open housing portion (2) extending distally therefrom, the partially open housing portion (2) is configured to cover the transmission drive train (21, 21') and its rotational transmission feature (27) within the housing assembly (10) when the surgical reamer driver (1) is in its assembled configuration.

15. The surgical reamer driver (1) of claim 14, wherein the partially open housing portion (2) is further configured to cover an elongated opening (30) on one side of the housing assembly (10) when the surgical reamer driver (1) is in its assembled configuration, the elongated opening (30) permitting removal of the transmission drive train (21, 21') for cleaning, and a further opening (111) on an opposite side of the housing assembly (10), the further opening (111) allowing a user's finger access to facilitate removal of the transmission drive train (21, 21').

16. The surgical reamer driver (1) of claim 1, wherein the handle assembly (9) is configured to transmit axial thrust into the housing assembly (10) and not into the transmission drive train (21, 21') or the motor shaft coupling (11, 11').

17. The surgical reamer driver (1) of claim 1, further comprising a release ring (7) disposed over the surgical tool connector (4) for disconnecting the surgical tool (227) from the surgical tool connector (4), the surgical tool connector (4) further including a locking head (190) adapted for coupling with at least one surgical tool (227), such locking head (190) being slidingly disposed and at least significantly enclosed within the center of the surgical reamer driver (1) and closely interfacing therewith so as to minimize seams, gaps or openings, and having an axial range of motion which assures that between an unlock and lock position, the locking head (190) is embedded in the surgical reamer driver (1) in the unlock position and is configured to then extend axially outwardly, in snug juxtaposition with the surgical reamer driver (1), to a lock position which is capable of locking a surgical tool (227) to the surgical reamer driver (1) in a manner so as to avoid the exposure of a significant seam, gap or opening during activation and so helping prevent debris and/or bone chips from entering the surgical reamer driver (1).

18. The surgical reamer driver (1) of claim 1 further including a sliding release sleeve (8), the sliding release sleeve (8) is configured to be lockable to and unlockable from the housing assembly (10) for assembly or disassembly, respectively.

19. The surgical reamer driver (1) of claim 18, wherein the housing assembly (1) has a slotted end (36') in which slots an annular locking feature (63), such as pins (63) of the handle assembly (9), engages and which is configured to be lockable to the handle (5) in a selected angular position wherein further a retainer, such as a snap feature, retains the handle (5) so as to prevent it from freely removing during disassembly.

20. The surgical reamer driver (1) of claim 19, wherein the retainer comprises at least one slot formed so that the slot (36') is smaller than an interfacing diameter of the annular locking feature (63), preferably a pin (63), a second slot (50) adjacent to the slot (36') forming a finger (51) between these slots (36', 50) and wherein, the finger may be elastically biased to expand an opening of the slot (36') to capture the angular locking feature (63).

21. A surgical kit (220) comprising the surgical reamer driver (1) of claim 1 together with other components, the kit (220) further comprising a case (221) for organizing and storing the components of the kit (220).

22. The surgical kit (220) of claim 21, further including:
a. surgical tools (227) of various sizes and styles, adapted to interface with the surgical tool connector (4);
b. optionally, an alternative motor shaft coupling (11, 11') having an alternative connection configuration; and
c. optionally, an alternate transmission drive train (21, 21') having an alternate surgical tool connector (4).

23. The surgical reamer driver (1) of claim 1, wherein the housing assembly (10) comprises an elongated bottom opening (30) facilitating disassembly.

## Patentansprüche

1. Chirurgischer Reibahlen-Antrieb (1), der ein distales und ein proximales Ende aufweist, wobei der chirurgische Reibahlen-Antrieb (1) aufweist:
(a) einen Gehäuseaufbau (10) in einer eigenständigen, aufgebauten Einheit, die eine proximale Öffnung (42) und eine distale Öffnung (28) aufweist,
(b) einen Übertragungsantriebsstrang (21, 21') in einer eigenständigen, aufgebauten Einheit, der in dem Gehäuseaufbau (10) einzuschließen ist und mindestens ein Doppelkreuzgelenk (22, 22'), das zwei angrenzende Kreuzgelenke umfasst, die mit Gabeln (130, 139) verbunden sind, und einen Verbinder für chirurgisches Werkzeug (4) an dem distalen Ende aufweist,
(c) eine Motorwellenkupplung (11, 11') in einer eigenständigen, aufgebauten Einheit, die an dem proximalen Ende davon anzuordnen ist, wobei die Motorwellenkupplung (11, 11') angepasst ist, um die proximale Öffnung (42) zu durchqueren, und rotierbar verbindbar und axial verschiebbar lösbar von dem Übertragungsantriebsstrang (21, 21') ist, und
(d) einen Griffaufbau (9) in einer eigenständigen, aufgebauten Einheit, die über dem Gehäuseaufbau (10) anzuordnen ist und lösbar von dem Gehäuseaufbau (10) ist,
wobei diese vier Grundkomponenten einen chirurgischen Reibahlen-Antrieb (1) ausbilden, der konfiguriert ist, um in einem vollständig aufgebauten Zustand Fremdkörper an einem Zugang zu dem Innenleben des chirurgischen Reibahlen-Antriebs (1) zu hindern.

2. Chirurgischer Reibahlen-Antrieb (1) nach Anspruch 1, wobei das mindestens eine Doppelkreuzgelenk (22, 22') des Übertragungsantriebsstrangs (21, 21') zwischen seiner Eingangs- und Ausgangswelle (24, 25) einen Winkel (70, 70') größer als 40° ausbildet.

3. Chirurgischer Reibahlen-Antrieb (1) nach Anspruch 1, wobei ein in Bezug auf den Gehäuseaufbau (10) feststehendes Lager (32) eine Flanschwelle (25) umgibt, die einen Flansch (33) aufweist, und wobei der Flansch (33) der Welle (25) gegen das Lager (32) wirkt, was eine axiale Bewegung der Welle (25) begrenzt.

4. Chirurgischer Reibahlen-Antrieb (1) nach Anspruch 1, wobei mindestens ein Abschnitt (139) der Gabeln (130, 139) der Doppelkreuzgelenke (22, 22') entfernt und in Bezug auf den anderen Abschnitt (130) der Gabeln wieder befestigt werden kann, um einen Aufbau zu erleichtern.

5. Chirurgischer Reibahlen-Antrieb (1) nach Anspruch 1, wobei mindestens eine der Gabeln (130, 139) der Doppelkreuzgelenke (22, 22') reibungsmindernde Lagerbüchsen (147, 148, 134, 134') umfasst.

6. Chirurgischer Reibahlen-Antrieb (1) nach Anspruch 5, wobei die Doppelkreuzgelenke (22, 22') die Lagerbüchsen (134, 134') aufweisen, die in mindestens einer Gabel (130, 139) aufgebaut sind, wodurch Reibung gemindert wird.

7. Chirurgischer Reibahlen-Antrieb (1) nach Anspruch 1, wobei der Griffaufbau (9) ein erstes Lager (114) enthält, das, wenn der Griffaufbau über dem den Antriebsstrang (21, 21') einschließenden Gehäuseaufbau (3, 10) aufgebaut ist, mit zweiten Lagern (115, 115') in dem Gehäuseaufbau (3, 10) zusammenwirkt, um den Antriebsstrang (21, 21') zu stabilisieren.

8. Chirurgischer Reibahlen-Antrieb (1) nach Anspruch 1, wobei das mindestens eine Doppelkreuzgelenk (22, 22') aus zwei angrenzenden Kreuzgelenkgestängen besteht, jedes Kreuzgelenkgestänge zwei Gabeln (131, 139, 130, 131') umfasst und die zwei Gabeln (131, 131'), die entlang der Übertragungsachse am weitesten beabstandet sind, zu 90° in Bezug aufeinander ausgerichtet sind.

9. Chirurgischer Reibahlen-Antrieb (1) nach Anspruch 1, wobei der Übertragungsantriebsstrang (21, 21') zwei Doppelkreuzgelenke (22, 22') umfasst, deren Eingangs- und Ausgangswelle (24, 25) in der aufgebauten Konfiguration einen Winkel (70, 70') größer als 40° ausbilden und wobei die Eingangswelle (25) des ersten Doppelkreuzgelenks (22, 22') versetzt und parallel zu der Ausgangswelle (24) des zweiten Doppelkreuzgelenks (22, 22') ist.

10. Chirurgischer Reibahlen-Antrieb (1) nach Anspruch 1, wobei die Motorwellenkupplung (11) an ihrem distalen Ende ein erstes Rotationsübertragungsmerkmal (29) umfasst, der Übertragungsantriebsstrang (21) an seinem proximalen Ende ein zweites Rotationsübertragungsmerkmal (27) umfasst, wobei in der aufgebauten Konfiguration das erste Rotationsübertragungsmerkmal (29) und das zweite Rotationsübertragungsmerkmal (27) derart axial verschiebbar und radial verbunden sind, dass eine Rotationsbewegung übertragen werden kann, wobei in der auseinandergebauten Konfiguration das erste Rotationsübertragungsmerkmal (29) und das zweite Rotationsübertragungsmerkmal (27) nicht verbunden sind und der Antriebsstrangaufbau (11, 21) in die Motorwellenkupplung (11) und den Übertragungsantriebsstrang (21) unterteilt ist, wobei in der aufgebauten Konfiguration die Rotationsübertragungsmerkmale (29, 27) eine Verbindung zwischen der Motorwellenkupplung (11) und dem chirurgischen Werkzeugverbinder (4) ausbilden und konfiguriert sind, um innerhalb des Gehäuseaufbaus (10) abgedeckt zu werden.

11. Chirurgischer Reibahlen-Antrieb (1) nach Anspruch 1, wobei der Griffaufbau (9) in dem aufgebauten Zustand über dem Gehäuseaufbau (10) derart angeordnet ist, dass die Motorwellenkupplung (11, 11') konfiguriert ist, um durch den Benutzer aufgebrachte Axialkräfte nicht auf den Übertragungsantriebsstrang (21, 21') zu übertragen und derartige Axialkräfte auf den chirurgischen Werkzeugverbinder (4) aufzubringen, und wobei der Griff (5) konfiguriert ist, um durch den Benutzer aufgebrachte Axialkräfte sowohl von dem Übertragungsantriebsstrang (21, 21') als auch von der Motorwellenkupplung (11, 11') zu entkuppeln und derartige Axialkräfte auf den chirurgischen Werkzeugverbinder (4) aufzubringen, vorzugsweise durch ein Drucklager.

12. Chirurgischer Reibahlen-Antrieb (1) nach Anspruch 1, wobei der chirurgische Werkzeugverbinder (4) an seinem distalen Ende eine zentrale Schneidwerkzeugverbindung (120) umfasst und die Motorwellenkupplung (11, 11') an ihrem proximalen Ende eine Motorwellenschnellverbindung (6) umfasst, wobei die Motorwellenschnellverbindung (6) der Motorwellenkupplung (11, 11') konfiguriert ist, um die zentrale Schneidwerkzeugverbindung (120) rotierbar mit einem Antriebsmotor zu verbinden.

13. Chirurgischer Reibahlen-Antrieb (1) nach Anspruch 1, wobei der chirurgische Reibahlen-Antrieb (1) derart konfiguriert ist, dass axiale Kräfte durch den Gehäuseaufbau (10) übertragbar sind, wobei dadurch der Übertragungsantriebsstrang (21, 21') umgangen wird, und, vorzugsweise über ein Lager (23), auf den chirurgischen Werkzeugverbinder (4) übertragen werden.

14. Chirurgischer Reibahlen-Antrieb (1) nach Anspruch 1, wobei der Übertragungsantriebsstrang (21) an seinem proximalen Ende ein Rotationsübertragungsmerkmal (27) umfasst, wobei die Griffaufbau (9) eine Freigabebüchse (8) und einen teilweise offenen Gehäuseabschnitt (2) enthält, der sich distal davon erstreckt, wobei der teilweise offene Gehäuseabschnitt (2) konfiguriert ist, um den Übertragungsantriebsstrang (21, 21') und dessen Rotationsübertragungsmerkmal (27) innerhalb des Gehäuseaufbaus (10) abzudecken, wenn sich der chirurgische Reibahlen-Antrieb (1) in seiner aufgebauten Konfiguration befindet.

15. Chirurgischer Reibahlen-Antrieb (1) nach Anspruch 14, wobei der teilweise offene Gehäuseabschnitt (2) ferner konfiguriert ist, um eine längliche Öffnung (30) auf einer Seite des Gehäuseaufbaus (10), wenn sich der chirurgische Reibahlen-Antrieb (1) in seiner aufgebauten Konfiguration befindet, wobei die längliche Öffnung (30) ein Entfernen des Übertragungsantriebsstrangs (21, 21') zum Reinigen zulässt, und eine weitere Öffnung (111) auf einer gegenüberliegenden Seite des Gehäuseaufbaus (10) abzudecken, wobei die weitere Öffnung (111) einem Finger eines Benutzers einen Zugang ermöglicht, um das Entfernen des Übertragungsantriebsstrangs (21, 21') zu erleichtern.

16. Chirurgischer Reibahlen-Antrieb (1) nach Anspruch 1, wobei der Griffaufbau (9) konfiguriert ist, um axialen Druck in den Gehäuseaufbau (10) und nicht in den Übertragungsantriebsstrang (21, 21') oder die Motorwellenkupplung (11, 11') zu übertragen.

17. Chirurgischer Reibahlen-Antrieb (1) nach Anspruch 1, ferner umfassend einen Freigabering (7), der über dem chirurgischen Werkzeugverbinder (4) zum Trennen des chirurgischen Werkzeugs (227) von dem Verbinder für chirurgisches Werkzeug (4) angeordnet ist, wobei der chirurgische Werkzeugverbinder (4) ferner einen Verriegelungskopf (190) enthält, der zum Kuppeln mit mindestens einem chirurgischen Werkzeug (227) angepasst ist, wobei ein derartiger Verriegelungskopf (190) verschiebbar angeordnet und mindestens wesentlich innerhalb des Zentrums des chirurgischen Reibahlen-Antriebs (1) eingeschlossen ist und eng an diesen anschließt, um Nähte, Spalten oder Öffnungen zu minimieren, und einen axialen Bewegungsbereich aufweist, der sicherstellt, dass der Verriegelungskopf (190) zwischen einer Entriegelungs- und einer Verriegelungsposition in der Entriegelungsposition in dem chirurgischen Reibahlen-Antrieb (1) eingebettet ist, und konfiguriert ist, um sich dann axial nach außen eng aneinander an dem chirurgischen Reibahlen-Antrieb (1) in eine Verriegelungsposition zu erstrecken, die in der Lage ist, um ein chirurgisches Werkzeug (227) mit dem chirurgischen Reibahlen-Antrieb (1) in einer Weise zu verriegeln, dass die Freilegung einer signifikanten Naht, eines Spalts oder einer Öffnung während einer Aktivierung vermieden wird, und so geholfen wird, dass Fremdkörper und/oder Knochenspäne an einem Eintreten in den chirurgischen Reibahlen-Antrieb (1) gehindert werden.

18. Chirurgischer Reibahlen-Antrieb (1) nach Anspruch 1, ferner enthaltend eine verschiebbare Freigabebuchse (8), wobei die verschiebbare Freigabebuchse (8) konfiguriert ist, um mit dem Gehäuseaufbau (10) zum Zusammenbau oder Auseinanderbau verriegelt beziehungsweise von diesem entriegelt zu werden.

19. Chirurgischer Reibahlen-Antrieb (1) nach Anspruch 18, wobei der Gehäuseaufbau (1) ein geschlitztes Ende (36') aufweist, in dessen Schlitze ein ringförmiges Verriegelungsmerkmal (63), wie zum Beispiel Stifte (63) des Griffaufbaus (9), eingreift und das konfiguriert ist, um mit dem Griff (5) in einer ausgewählten Winkelposition verriegelbar zu sein, wobei ferner ein Halter, wie zum Beispiel ein Schnappmerkmal, den Griff (5) hält, um ihn während des Auseinanderbaus am freien Entfernen zu hindern.

20. Chirurgischer Reibahlen-Antrieb (1) nach Anspruch 19, wobei der Halter mindestens einen Schlitz umfasst, der so ausgebildet ist, dass der Schlitz (36') kleiner ist als ein Anschließdurchmesser des ringförmigen Verriegelungsmerkmals (63), vorzugsweise ein Stift (63), ein zweiter Schlitz (50), der an den Schlitz (36') angrenzt, einen Finger (51) zwischen diesen Schlitzen (36', 50) ausbildet und wobei der Finger elastisch vorgespannt sein kann, um eine Öffnung des Schlitzes (36') zu erweitern, um das winkelförmige Verriegelungsmerkmal (63) zu erfassen.

21. Chirurgisches Kit (220), das den chirurgischen Reibahlen-Antrieb (1) nach Anspruch 1 zusammen mit anderen Komponenten umfasst, wobei das Kit (220) ferner einen Koffer (221) zum Organisieren und Aufbewahren der Komponenten des Kits (220) umfasst.

22. Chirurgisches Kit (220) nach Anspruch 21, das ferner enthält:
a. chirurgische Werkzeuge (227) verschiedener Größen und Ausführungen, die angepasst sind, um an den chirurgischen Werkzeugverbinder (4) anzuschließen;
b. optional eine alternative Motorwellenkupplung (11, 11'), die eine alternative Verbindungskonfiguration aufweist; und
c. optional einen alternativen Übertragungsantriebsstrang (21, 21'), der einen alternativen chirurgisches Werkzeugverbinder (4) aufweist.

23. Chirurgischer Reibahlen-Antrieb (1) nach Anspruch 1, wobei der Gehäuseaufbau (10) eine längliche untere Öffnung (30) umfasst, die den Auseinanderbau erleichtert.

## Revendications

1. Dispositif d'entraînement d'alésoir chirurgical (1) ayant une extrémité distale et proximale, le dispositif d'entraînement d'alésoir chirurgical (1) ayant :
(a) un ensemble boîtier (10) dans une unité assemblée autonome ayant une ouverture proximale (42) et une ouverture distale (28),
(b) une chaîne cinématique de transmission (21, 21') dans une unité assemblée autonome devant être enfermée dans l'ensemble boîtier (10), et ayant au moins un double joint universel (22, 22') comprenant deux joints universels adjacents reliés par des fourches (130, 139) et un raccord d'outil chirurgical (4) au niveau de l'extrémité distale,
(c) un accouplement d'arbre de moteur (11, 11') dans une unité assemblée autonome devant être disposé au niveau de son extrémité proximale, l'accouplement d'arbre de moteur (11, 11') étant adapté pour passer à travers l'ouverture proximale (42) et pouvant être relié en rotation et pouvant être détaché axialement de manière coulissante de la chaîne cinématique de transmission (21, 21'), et
(d) un ensemble poignée (9) dans une unité assemblée autonome devant être disposé au-dessus de l'ensemble boîtier (10) et pouvant être détaché de l'ensemble boîtier (10), ces quatre composants de base formant un dispositif d'entraînement d'alésoir chirurgical (1) conçu pour empêcher efficacement, dans un état entièrement assemblé, les débris d'accès au fonctionnement interne du dispositif d'entraînement d'alésoir chirurgical (1).

2. Dispositif d'entraînement d'alésoir chirurgical (1) selon la revendication 1, dans lequel l'au moins un double joint universel (22, 22') de la chaîne cinématique de transmission (21, 21') forme un angle (70, 70') supérieur à 40° entre ses arbres d'entrée et de sortie (24, 25).

3. Dispositif d'entraînement d'alésoir chirurgical (1) selon la revendication 1, dans lequel un palier (32), fixé par rapport à l'ensemble boîtier (10) entoure un arbre à bride (25) ayant une bride (33), et dans lequel la bride (33) de l'arbre (25) agit contre le palier (32) limitant le mouvement axial de l'arbre (25).

4. Dispositif d'entraînement d'alésoir chirurgical (1) selon la revendication 1, dans lequel au moins une partie (139) des fourches (130, 139) des doubles joints universels (22, 22') peut être retirée et refixée par rapport à l'autre partie (130) des fourches pour faciliter l'assemblage.

5. Dispositif d'entraînement d'alésoir chirurgical (1) selon la revendication 1, dans lequel au moins une des fourches (130, 139) des doubles joints universels (22, 22') comprend des manchons de paliers réducteurs de frottement (147, 148, 134, 134').

6. Dispositif d'entraînement d'alésoir chirurgical (1) selon la revendication 5, dans lequel les doubles joints universels (22, 22') ont les manchons de palier (134, 134') assemblés dans au moins une fourche (130, 139) réduisant ainsi le frottement.

7. Dispositif d'entraînement d'alésoir chirurgical (1) selon la revendication 1, dans lequel l'ensemble poignée (9) comporte un premier palier (114) qui, lorsque l'ensemble poignée est assemblé sur l'ensemble boîtier (3, 10) enfermant la chaîne cinématique (21, 21'), coopère avec les seconds paliers (115, 115') dans l'ensemble boîtier (3, 10) pour stabiliser la chaîne cinématique (21, 21').

8. Dispositif d'entraînement d'alésoir chirurgical (1) selon la revendication 1, dans lequel l'au moins un double joint universel (22, 22') est constitué de deux liaisons de joint universel adjacentes, chaque liaison de joint universel comprend deux fourches (131, 139, 130, 131), et les deux fourches (131, 131') les plus espacées le long de l'axe de transmission sont orientées à 90° l'une par rapport à l'autre.

9. Dispositif d'entraînement d'alésoir chirurgical (1) selon la revendication 1, dans lequel la chaîne cinématique de transmission (21, 21') comprend deux doubles joints universels (22, 22') ayant leurs arbres d'entrée et de sortie (24, 25) qui forment dans la configuration assemblée un angle (70, 70') supérieur à 40° et dans lequel l'arbre d'entrée (25) du premier double joint universel (22, 22') est décalé et parallèle à l'arbre de sortie (24) du second double joint universel (22, 22').

10. Dispositif d'entraînement d'alésoir chirurgical (1) selon la revendication 1, dans lequel l'accouplement d'arbre de moteur (11) comprend un premier élément de transmission rotatif (29) sur son extrémité distale, la chaîne cinématique de transmission (21) comprend un second élément de transmission rotatif (27) sur son extrémité proximale, dans lequel, dans la configuration assemblée, le premier élément de transmission rotatif (29) et le second élément de transmission rotatif (27) peuvent coulisser axialement et sont reliés radialement de sorte qu'un mouvement de rotation peut être transmis, dans lequel, dans la configuration désassemblée, le premier élément de transmission rotatif (29) et le second élément de transmission rotatif (27) ne sont pas reliés et l'ensemble chaîne cinématique (11, 21) est divisé en accouplement d'arbre de moteur (11) et en chaîne cinématique de transmission (21), dans lequel, dans la configuration assemblée, les éléments de transmission rotatifs (29, 27) forment une liaison entre l'accouplement d'arbre de moteur (11) et le raccord d'outil chirurgical (4) et sont conçus pour être recouverts à l'intérieur de l'ensemble de boîtier (10).

11. Dispositif d'entraînement d'alésoir chirurgical (1) selon la revendication 1, dans lequel l'ensemble poignée (9) est disposé sur l'ensemble boîtier (10) dans l'état assemblé de sorte que l'accouplement d'arbre de moteur (11, 11') est conçu pour ne pas transmettre de forces axiales appliquées par l'utilisateur à la chaîne cinématique de transmission (21, 21'), et pour appliquer de telles forces axiales au raccord d'outil chirurgical (4) et dans lequel la poignée (5) est configurée pour découpler les forces axiales appliquées par l'utilisateur à la fois de la chaîne cinématique de transmission (21, 21') et de l'accouplement d'arbre de moteur (11, 11') et pour appliquer de telles forces axiales au raccord d'outil chirurgical (4), de préférence par l'intermédiaire d'un palier de butée.

12. Dispositif d'entraînement d'alésoir chirurgical (1) selon la revendication 1, dans lequel le raccord d'outil chirurgical (4) comprend un raccordement d'outil de coupe central (120) au niveau de son extrémité distale et l'accouplement d'arbre de moteur (11, 11') comprend un raccordement rapide d'arbre de moteur (6) au niveau de son extrémité proximale, dans lequel le raccordement rapide d'arbre de moteur (6) de l'accouplement d'arbre de moteur (11, 11') est conçu pour relier de manière rotative le raccordement d'outil de coupe central (120) à un moteur d'entraînement.

13. Dispositif d'entraînement d'alésoir chirurgical (1) selon la revendication 1, dans lequel le dispositif d'entraînement d'alésoir chirurgical (1) est conçu de sorte que des forces axiales peuvent être transmises à travers l'ensemble boîtier (10) contournant ainsi la chaîne cinématique de transmission (21, 21'), et sont transmises, de préférence par l'intermédiaire d'un palier (23), au raccord d'outil chirurgical (4).

14. Dispositif d'entraînement d'alésoir chirurgical (1) selon la revendication 1, dans lequel la chaîne cinématique de transmission (21) comprend un élément de transmission rotatif (27) sur son extrémité proximale, dans lequel l'ensemble poignée (9) comporte un manchon de libération (8) et une partie de boîtier partiellement ouverte (2) s'étendant distalement à partir de celle-ci, la partie de boîtier partiellement ouverte (2) est conçue pour recouvrir la chaîne cinématique de transmission (21, 21') et son élément de transmission rotatif (27) à l'intérieur de l'ensemble boîtier (10) lorsque le dispositif d'entraînement d'alésoir chirurgical (1) est dans sa configuration assemblée.

15. Dispositif d'entraînement d'alésoir chirurgical (1) selon la revendication 14, dans lequel la partie de boîtier partiellement ouverte (2) est en outre conçue pour couvrir une ouverture allongée (30) sur un côté de l'ensemble boîtier (10) lorsque le dispositif d'entraînement d'alésoir chirurgical (1) est dans sa configuration assemblée, l'ouverture allongée (30) permettant le retrait de la chaîne cinématique de transmission (21, 21') en vue de son nettoyage, et une autre ouverture (111) sur un côté opposé de l'ensemble boîtier (10), l'autre ouverture (111) permettant l'accès du doigt d'un utilisateur pour faciliter le retrait de la chaîne cinématique de transmission (21, 21').

16. Dispositif d'entraînement d'alésoir chirurgical (1) selon la revendication 1, dans lequel l'ensemble poignée (9) est conçu pour transmettre une poussée axiale dans l'ensemble boîtier (10) et non dans la chaîne cinématique de transmission (21, 21') ou l'accouplement d'arbre de moteur (11, 11').

17. Dispositif d'entraînement d'alésoir chirurgical (1) selon la revendication 1, comprenant en outre une bague de libération (7) disposée sur le raccord d'outil chirurgical (4) pour séparer l'outil chirurgical (227) du raccord d'outil chirurgical (4), le raccord d'outil chirurgical (4) comportant en outre une tête de verrouillage (190) adaptée pour être accouplée avec au moins un outil chirurgical (227), cette tête de verrouillage (190) étant disposée de manière coulissante et au moins sensiblement enfermée au centre du dispositif d'entraînement d'alésoir chirurgical (1) et couplée étroitement avec celui-ci de manière à réduire le plus possible les coutures, les espaces ou les ouvertures, et ayant une plage de mouvement axial qui garantit qu'entre une position de déverrouillage et de verrouillage, la tête de verrouillage (190) est intégrée dans le dispositif d'entraînement d'alésoir chirurgical (1) dans la position de déverrouillage et est conçue pour s'étendre ensuite axialement vers l'extérieur, en juxtaposition serrée avec le dispositif d'entraînement d'alésoir chirurgical (1), jusqu'à une position de verrouillage qui est capable de verrouiller un outil chirurgical (227) sur le dispositif d'entraînement d'alésoir chirurgical (1) de manière à éviter l'exposition d'une couture, d'un espace ou d'une ouverture importants lors de l'activation et contribuant à empêcher ainsi la pénétration de débris et/ou de copeaux osseux dans le dispositif d'entraînement d'alésoir chirurgical (1).

18. Dispositif d'entraînement d'alésoir chirurgical (1) selon la revendication 1, comportant en outre un manchon de libération coulissant (8), le manchon de libération coulissant (8) étant conçu pour pouvoir être verrouillé et déverrouillé de l'ensemble boîtier (10) pour assemblage ou désassemblage, respectivement.

19. Dispositif d'entraînement d'alésoir chirurgical (1) selon la revendication 18, dans lequel l'ensemble boîtier (1) a une extrémité à fentes (36') dans lesquelles fentes un élément de verrouillage annulaire (63), tel que des broches (63) de l'ensemble poignée (9), vient en prise et qui est conçu pour pouvoir être verrouillé à la poignée (5) dans une position angulaire sélectionnée, dans lequel en outre un élément de retenue, tel qu'un élément d'encliquetage, retient la poignée (5) de manière à l'empêcher de se retirer librement pendant le désassemblage.

20. Dispositif d'entraînement d'alésoir chirurgical (1) selon la revendication 19, dans lequel le dispositif de retenue comprend au moins une fente formée de sorte que la fente (36') est plus petite qu'un diamètre de couplage de l'élément de verrouillage annulaire (63), de préférence une broche (63), une seconde fente (50) adjacente à la fente (36') formant un doigt (51) entre ces fentes (36', 50) et dans lequel le doigt peut être sollicité élastiquement pour dilater une ouverture de la fente (36') pour capturer l'élément de verrouillage angulaire (63).

21. Kit chirurgical (220) comprenant le dispositif d'entraînement d'alésoir chirurgical (1) selon la revendication 1 ainsi que d'autres composants, le kit (220) comprenant en outre un étui (221) pour ranger et stocker les composants du kit (220).

22. Kit chirurgical (220) selon la revendication 21, comportant en outre :
a. des outils chirurgicaux (227) de tailles et de types variés, adaptés pour être reliés par interface au raccord d'outil chirurgical (4) ;
b. facultativement, un accouplement d'arbre de moteur (11, 11') alternatif ayant une configuration de raccordement alternative ; et
c. facultativement, une chaîne cinématique de transmission (21, 21') alternative ayant un autre raccord d'outil chirurgical (4).

23. Dispositif d'entraînement d'alésoir chirurgical (1) selon la revendication 1, dans lequel l'ensemble boîtier (10) comprend une ouverture inférieure allongée (30) facilitant le désassemblage.
